# EUROPEAN PATENT SPECIFICATION

(11) **EP 3 665 650 B1**
(45) Date of publication and mention of the grant of the patent: **20.10.2021**
(21) Application number: 18758675.5
(22) Date of filing: 07.08.2018
(51) Int. Cl.: G06T 7/00, G06T 7/521, G06T 7/593

(54) **METHOD AND APPARATUS FOR MEASURING THE ACCURACY OF MODELS GENERATED BY A PATIENT MONITORING SYSTEM**
VERFAHREN UND VORRICHTUNG ZUR MESSUNG DER GENAUIGKEIT VON MODELLEN, DIE VON EINEM PATIENTENÜBERWACHUNGSSYSTEM ERZEUGT WERDEN
PROCÉDÉ ET APPAREIL SERVANT À MESURER LA PRÉCISION DE MODÈLES GÉNÉRÉS PAR UN SYSTÈME DE SURVEILLANCE DE PATIENT

(30) Priority: 08.08.2017 GB 201712721
(43) Date of publication of application: 17.06.2020
(73) Proprietor: Vision RT Limited, London, Greater London N3 2JU (GB)
(72) Inventor: MEIR, Ivan Daniel, London Greater London N3 2JU (GB); BARRETT, Adrian Roger William, London Greater London N3 2JU (GB)
(74) Representative: Demant
(86) International application number: PCT/GB2018/052248
(87) International publication number: WO 2019/030512

(56) References cited:
- EP-A1- 3 093 824
- MATTHEW BERGER ET AL: "A benchmark for surface reconstruction", ACM TRANSACTIONS ON GRAPHICS (TOG), ACM, US, vol. 32, no. 2, 30 April 2013 (2013-04-30) , pages 1-17, XP058042516, ISSN: 0730-0301, DOI: 10.1145/2451236.2451246
- ESDRAS MEDEIROS ET AL: "Using physically Based Rendering to Benchmark Structured Light Scanners", COMPUTER GRAPHICS FORUM, vol. 33, no. 7, 1 October 2014 (2014-10-01), pages 71-80, XP055374169, GB ISSN: 0167-7055, DOI: 10.1111/cgf.12475
- ADAM CHROMY: "Abstract", INTERNATIONAL JOURNAL OF ELECTRONICS AND TELECOMMUNICATIONS, vol. 62, no. 1, 1 March 2016 (2016-03-01), pages 23-31, XP055514084, DOI: 10.1515/eletel-2016-0003
- Dong-Min Woo ET AL: "Performance of Correlation-Based Stereo Algorithm with Respect to the Change of the Window Size" In: "Medical image computing and computer-assisted intervention - MICCAI 2015 : 18th international conference, Munich, Germany, October 5-9, 2015; proceedings", 1 January 2004 (2004-01-01), Springer International Publishing, Cham 032548, XP055514089, ISSN: 0302-9743 ISBN: 978-3-319-24946-9 vol. 3332, pages 778-785, DOI: 10.1007/978-3-540-30542-2_96, abstract page 781, paragraph 2

## Description

The present invention relates to methods and apparatus for measuring the accuracy of models generated by a patient monitoring system. More particularly, embodiments of the present invention relate to measuring the accuracy of models generated by patient monitoring systems which generate models of the surface of a patient on the basis of captured images of a patient on to which a pattern of light is projected. The invention is particularly suitable for use with monitoring systems for monitoring patient positioning during radiation therapy and the like where highly accurate positioning and the detection of patient movement is important for successful treatment.

Radiotherapy consists of projecting onto a predetermined region of a patient's body, a radiation beam so as to destroy or eliminate tumors existing therein. Such treatment is usually carried out periodically and repeatedly. At each medical intervention, the radiation source must be positioned with respect to the patient in order to irradiate the selected region with the highest possible accuracy to avoid radiating adjacent tissue on which radiation beams would be harmful. For this reason, a number of monitoring systems for assisting the positioning of patients during radiotherapy have been proposed such as those described in Vision RT's earlier patents and patent applications in U.S. Pat. Nos. 7,889,906, 7,348,974, 8,135,201, 9,028,422, and US Pat Application Nos. 2015/265852 and 2016/129283, all of which are hereby incorporated by reference.

In Vision RT's monitoring system, a speckled pattern of light is projected onto the surface of a patient to facilitate identification of corresponding portions of the surface of a patient captured from different viewpoints. Images of a patient are obtained and processed together with data identifying the relative locations of the cameras capturing the images to identify 3D positions of a large number of points corresponding to points on the surface of a patient. Such data can be compared with data generated on a previous occasion and used to position a patient in a consistent manner or provide a warning when a patient moves out of position. Typically such a comparison involves undertaking Procrustes analysis to determine a transformation which minimizes the differences in position between points on the surface of a patient identified by data generated based on live images and points on the surface of a patient identified by data generated on a previous occasion. Other radio therapy patient monitoring systems monitor the position of patients undergoing radiotherapy by projecting structured light (e.g. laser light) in the form of a line or grid pattern or other predefined pattern onto the surface of a patient and generating a model of the surface on a patient being monitored on the basis of the appearance of the projected pattern in captured images. The accuracy of models generated by patient monitoring systems are subject to various factors. In many systems, the accuracy of models is dependent upon the angle at which a patient is viewed. If a portion of a patient is viewed at an oblique angle, the accuracy of a generated model is often lower than for portions of a patient viewed at a less oblique angle. Many monitoring systems attempt to alleviate this problem by capturing images of a patient from multiple angles. If multiple cameras are utilized there is a greater chance that the view of any particular camera might be blocked as the radiation treatment apparatus moves during the course of treatment. If the view of a particular camera is blocked, it may be possible to model a part of the surface of a patient using image data from another camera. However, when this occurs the generated model may not exactly correspond to previously generated models and this can erroneously be taken to indicate that a patient has moved during treatment which may cause an operator to halt treatment until a patient can be repositioned.

It would be helpful to be able to identify when in the course of treatment such erroneous detections of movement might occur.

More generally, it would be helpful to be able to identify the reliability and accuracy of generated models of the surface of a patient and how that accuracy varies during the course of a treatment plan.

The articles "A benchmark for surface reconstruction" by M. Berger and "Using physically Based Rendering to Benchmark Structured Light Scanners" by E. Medeiros disclose methods where synthetic images corresponding to different structured light scanning techniques applied onto an object test model are simulated. The then obtained synthetic images are used to retrieve the object surface which is finally compared to the object test model to estimate errors which arise when modelling a surface of an object using a light scanner.

In accordance with one aspect of the present invention there is provided a method of measuring the accuracy of a model of a patient generated by a patient monitoring system operable to generate a model of a patient on the basis of images of patterns of light projected onto the surface of a patient, as defined in claim 1.

In such a system a simulated image of a surface of a patient onto which a pattern of light is projected by texture rendering a model of a surface of a patient is generated. The simulated image is then processed so as to generate a model of the surface of a patient and the generated model is then compared with the model utilized to generate the simulated image. The extent to which the generated model and the model utilized to generate the simulated image differ is indicative of the errors which arise when modelling a surface of a patient using the modelling system to monitor a patient during treatment.

In some embodiments generating a simulated image of a surface of a patient onto which a pattern of light is projected by texture rendering a model of a surface of a patient may comprise generating a plurality of simulated images of a surface of a patient onto which a pattern of light is projected by texture rendering a model of a surface of a patient. The plurality of images may comprise simulated images of a surface of a patient onto which a pattern of light has been projected is viewed from a plurality of different view points and processing the simulated images to generate a model of the surface of a patient may comprise processing the simulated images to identify corresponding portions of the simulated surface of a patient onto which a pattern of light is projected appearing in the simulated images.

In some embodiments the simulated pattern of light may comprise a speckled pattern of light. In other embodiments the simulated pattern of light may comprise a pattern of structured light such a grid pattern or a line of laser light or other predefined pattern of light. In some embodiments generating a simulated image of a surface of a patient onto which a pattern of light is projected may further comprise generating a model of a treatment apparatus positioned in accordance with positioning instructions wherein generating a simulated image of a surface of a patient onto which a pattern of light is projected comprises generating a simulated image of a surface of a patient and the treatment apparatus positioned in accordance with positioning instructions onto which a pattern of light is projected.

In some embodiments comparing a generated model with a model utilized to generate a simulated image may comprise determining an offset value indicative of an implied distance between a generated model and a model utilized to generate a simulated image.

In some embodiments generating a model of the surface of a patient may comprise selecting a portion of one or more simulated images to be processed to generate a model of the surface of a patient.

In some embodiments processing simulated images to generate a model of the surface of a patient may comprise manipulating the simulated images to represent image and/or lens distortions and/or camera mis-calibrations.

In some embodiments comparisons between generated models and models utilized to generate simulated images may be utilized to identify how the accuracy of a model varies based upon movement of a treatment apparatus and/or repositioning of a patient during the course of a treatment session.

In the invention comparisons between generated models and models utilized to generate simulated images may be utilized to select portions of images to be used to monitor a patient for patient motion during treatment.

Further aspects of the present invention provide a simulation apparatus operable to measure the accuracy of models generated by a patient monitoring system and a computer readable medium operable to cause a programmable computer to perform a method of measuring the accuracy of a model of a patient generated by a patient monitoring system as described above.

Embodiments of the present invention will now be described in greater detail with reference to the accompanying drawings in which:
Figure 1 is a schematic perspective view of a treatment apparatus and a patient monitor;
Figure 2 is a front perspective view of a camera pod of the patient monitor of figure 1;
Figure 3 is a schematic block diagram of the computer system of the patient monitor of figure 1;
Figure 4 is a schematic block diagram of a simulation apparatus in accordance with an embodiment of the present invention;
Figure 5 is a flow diagram of the processing undertaken by the simulation apparatus of Figure 4; and
Figure 6 is a schematic illustration of a simulated image of a modelled surface of a patient and a treatment apparatus.

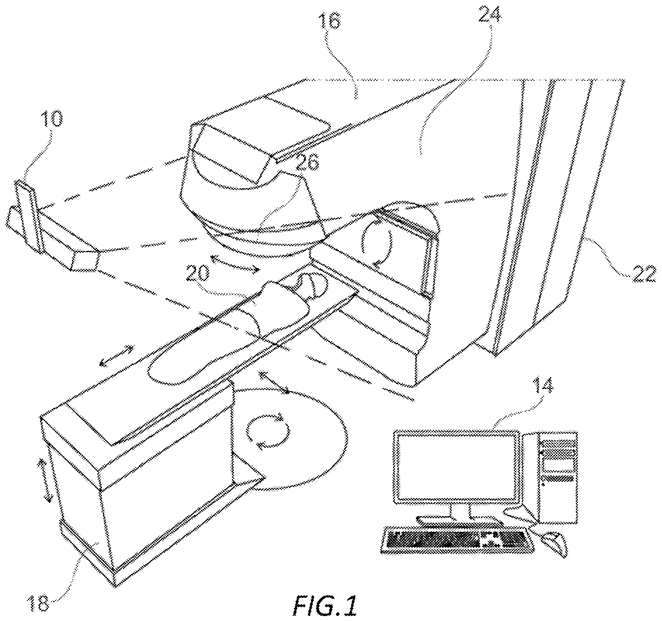

Prior to describing a method of measuring the accuracy of a model of a patient generated by a patient modelling system, an exemplary patient monitoring system and radiotherapy treatment apparatus images of which might be simulated will first be described with reference to Figures 1-3.

Figure 1 is a schematic perspective view of an exemplary patient monitoring system comprising a camera system comprising a number of cameras mounted within a number of camera pods 10 one of which is shown in Figure 1 that are connected by wiring (not shown) to a computer 14. The computer 14 is also connected to treatment apparatus 16 such as a linear accelerator for applying radiotherapy. A mechanical couch 18 is provided as part of the treatment apparatus upon which a patient 20 lies during treatment. The treatment apparatus 16 and the mechanical couch 18 are arranged such that, under the control of the computer 14, the relative positions of the mechanical couch 18 and the treatment apparatus 16 may be varied, laterally, vertically, longitudinally and rotationally as is indicated in the figure by the arrows adjacent the couch. In some embodiments the mechanical couch 18 may additionally be able to adjust the pitch, yaw and roll of a patient 20 as well.

The treatment apparatus 16 comprises a main body 22 from which extends a gantry 24. A collimator 26 is provided at the end of the gantry 24 remote from the main body 22 of the treatment apparatus 16. To vary the angles at which radiation irradiates a patient 20, the gantry 24, under the control of the computer 14, is arranged to rotate about an axis passing through the center of the main body 22 of the treatment apparatus 16 as indicated on the figure. Additionally the direction of irradiation by the treatment apparatus may also be varied by rotating the collimator 26 at the end of the gantry 24 as also indicated by the arrows on the figure.

To obtain a reasonable field of view in a patient monitoring system, cameras pods 10 containing cameras monitoring a patient 20, typically view a patient 20 from a distance (e.g. 1 to 2 meters from the patient being monitored). In the exemplary illustration of Figure 1, the field of view of the camera pod 10 shown in Figure 1 is indicated by the dashed lines extending away from the camera pod 10.

As is shown in Figure 1, typically such camera pods 10 are suspended from the ceiling of a treatment room and are located away from the gantry 26 so that the camera pods 10 do not interfere with the rotation of the gantry 26. In some systems a camera system including only a single camera pod 10 is utilized. However, in other systems, it is preferable for the camera system to include multiple camera pods 10 as rotation of the gantry 26 may block the view of a patient 20 in whole or in part when the gantry 26 or the mechanical couch 18 are in particular orientations. The provision of multiple camera pods 10 also facilitates imaging a patient from multiple directions which may increase the accuracy of the system.

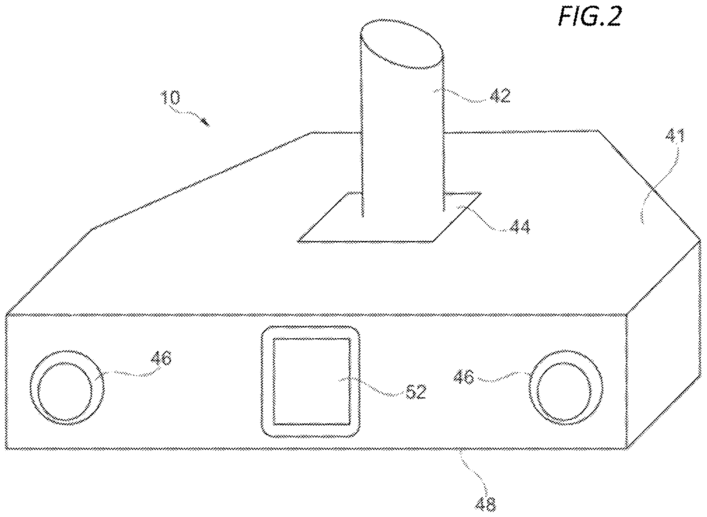

Figure 2 is a front perspective view of an exemplary camera pod 10.

The camera pod 10 in this example comprises a housing 41 which is connected to a bracket 42 via a hinge 44. The bracket 42 enables the camera pod 10 to be attached in a fixed location to the ceiling of a treatment room whilst the hinge 44 permits the orientation of the camera pod 10 to be orientated relative to the bracket 42 so that the camera pod 10 can be arranged to view a patient 20 on a mechanical couch 18. A pair of lenses 46 are mounted at either end of the front surface 48 of the housing 41. These lenses 46 are positioned in front of image capture devices/cameras such as CMOS active pixel sensors or charge coupled devices (not shown) contained within the housing 41. The cameras/image detectors are arranged behind the lenses 46 so as to capture images of a patient 20 via the lenses 46.

In this example, a speckle projector 52 is provided in the middle of the front surface 48 of the housing 41 between the two lenses 46 in the camera pod 10 shown in Figure 2. The speckle projector 52 in this example is arranged to illuminate a patient 20 with a non-repeating speckled pattern of red light so that when images of a patient 20 are captured by the two image detectors mounted within a camera pod 10 corresponding portions of captured images can be more easily distinguished. To that end the speckle projector comprises a light source such as a LED and a film with a random speckle pattern printed on the film. In use light from the light source is projected via the film and as a result a pattern consisting of light and dark areas is projected onto the surface of a patient 20. In some monitoring systems, the speckle projector 52 could be replaced with a projector arranged to project structured light (e.g. laser light) in the form of a line or a grid pattern onto the surface of a patient 20.

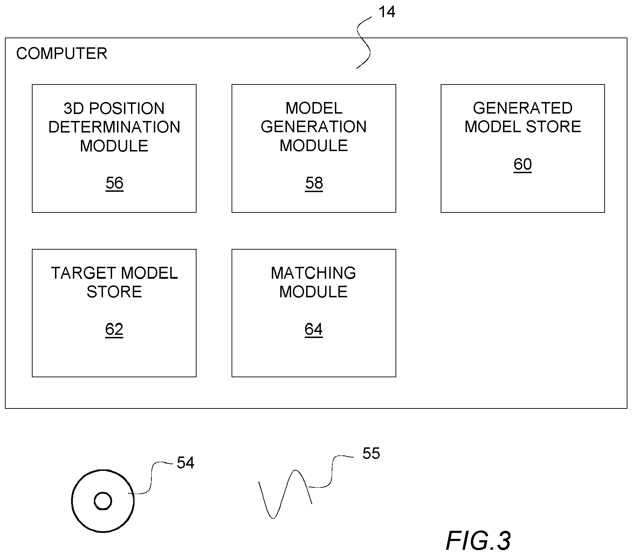

Figure 3 is a schematic block diagram of the computer 14 of the patient monitor of Figure 1. In order for the computer 14 to process images received from the camera pods 10, the computer 14 is configured by software either provided on a disk 54 or by receiving an electrical signal 55 via a communications network into a number of functional modules 56-64. In this example, the functional modules 56-64 comprise: a 3D position determination module 56 for processing images received from the stereoscopic camera system 10; a model generation module 58 for processing data generated by the 3D position determination module 56 and converting the data into a 3D wire mesh model of an imaged computer surface; a generated model store 60 for storing a 3D wire mesh model of an imaged surface; a target model store 62 for storing a previously generated 3D wire mesh model; and a matching module 64 for determining rotations and translations required to match a generated model with a target model.

In use, as images are obtained by the image capture devices/cameras of the camera pods 10, these images are processed by the 3D position determination module 56. This processing enables the 3D position determination module to identify 3D positions of corresponding points in pairs of images on the surface of a patient 20. In the exemplary system, this is achieved by the 3D position determination module 56 identifying corresponding points in pairs of images obtained by the camera pods 10 and then determining 3D positions for those points based on the relative positions of corresponding points in obtained pairs of images and stored camera parameters for each of the image capture devices/cameras of the camera pods 10.

The position data generated by the 3D position determination module 56 is then passed to the model generation module 58 which processes the position data to generate a 3D wire mesh model of the surface of a patient 20 imaged by the stereoscopic cameras 10. The 3D model comprises a triangulated wire mesh model where the vertices of the model correspond to the 3D positions determined by the 3D position determination module 56. When such a model has been determined it is stored in the generated model store 60.

In other systems such as those based on the projection of structured light onto the surface of a patient, rather than processing pairs of images to identify corresponding points in pairs of images on the surface of a patient, a model of the surface of a patient is generated based upon the manner in which the pattern of structured light appears in images.

When a wire mesh model of the surface of a patient 20 has been stored, the matching module 64 is then invoked to determine a matching translation and rotation between the generated model based on the current images being obtained by the stereoscopic cameras 10 and a previously generated model surface of the patient stored in the target model store 62. The determined translation and rotation can then be sent as instructions to the mechanical couch 18 to cause the couch to position the patient 20 in the same position relative to the treatment apparatus 16 as the patient 20 was when the patient 20 was previously treated.

Subsequently, the image capture devices/cameras of the camera pods 10 can continue to monitor the patient 20 and any variation in position can be identified by generating further model surfaces and comparing those generated surfaces with the target model stored in the target model store 62. If it is determined that a patient 20 has moved out of position, the treatment apparatus 16 can be halted or a warning can be triggered and the patient 20 repositioned, thereby avoiding irradiating the wrong parts of the patient 20.

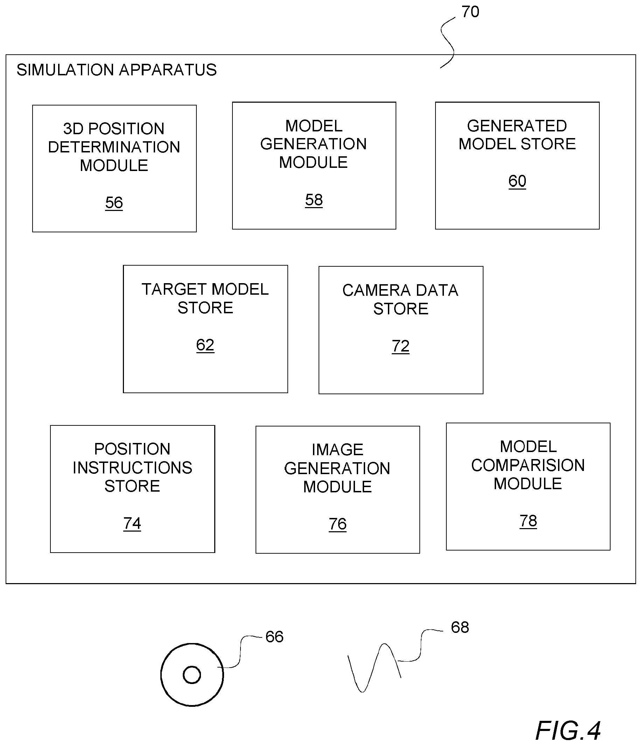

Figure 4, is a schematic block diagram of a simulation apparatus 70 in accordance with an embodiment of the present invention. As with the computer 14 of the patient monitor, the simulation apparatus 70 comprises a computer configured by software either provided on a disk 66 or by receiving an electrical signal 68 via a communications network into a number of functional modules 56-62, 74-78 which in this embodiment comprise: a 3D position determination module 56; a model generation module 58; a generated model store 60; and a target model store 62 identical to those in the computer 14 of the patient monitor along with a camera data store 72, a position instructions store 74, an image generation module 76 and a model comparison module 78.

In this embodiment the camera data store 72 stores data identifying the locations of cameras of a monitoring system; the position instructions store 74 stores data identifying the orientations the treatment apparatus 16 and couch 18 are instructed to adopt during treatment; and the image generation module 76 is a module arranged to process data within the target model store 62, camera data store 72 and position instructions store 74 to generate simulated images of the surface of a patient onto which a pattern of light is projected; and the model comparison module 78 is operable to compare model surfaces generated by the model generation module 58 with the target model stored in the target model store 62.

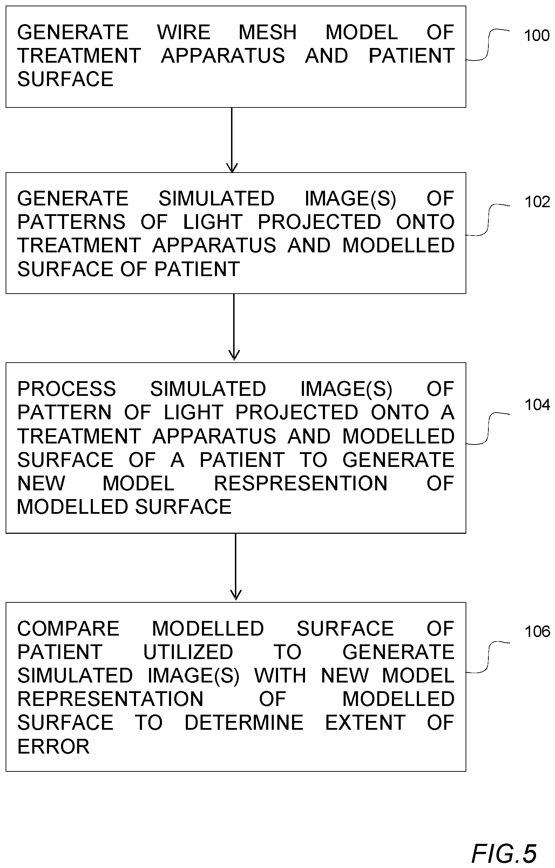

The processing undertaken by the simulation apparatus 70 is illustrated in the flow diagram shown in Figure 5.

As an initial step 100, the image generation module 76 generates a wire mesh model of a treatment apparatus and a patient surface positioned on the basis of instructions in the position instruction store 74 and the target model store 62. In the case of the model of the treatment apparatus 16 this will be a predefined representation of the treatment apparatus 16 and mechanical couch 18 used to position and treat a patient 20 where the positions and orientations of the modelled treatment apparatus 16 and mechanical couch 18 correspond to the position and orientation identified by position instructions for the position and orientation being modelled. In the case of modelling the surface of a patient the model of the patient stored in the target model store 62 is utilized with the model being rotated about the treatment room iso-center in the manner identified by the orientation of the mechanical couch 18 as identified by the position instructions for the position and orientation being modelled.

Having generated this wire mesh model of the surface of a patient 20 and a treatment apparatus 16 and couch 18 in a particular orientation, the image generation module 76 then proceeds 102 to generate a number of texture rendered images of the wire mesh model from viewpoints corresponding to the camera positions identified by data in the camera data store 72.

The generation of such simulated images is achieved in a conventional way using texture render software. In the images, in this embodiment a speckle pattern corresponding to the speckle pattern projected by the speckle projectors 52 of the camera pods is used to texture render the generated wire mesh model.

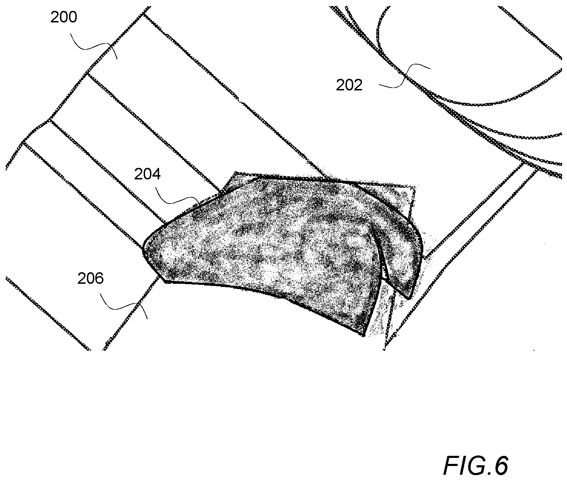

A schematic illustration of a simulated image of a modelled surface of a patient and a treatment apparatus is shown in Figure 6.

In the illustrated example the model of the treatment apparatus 200 is shown in a position where the apparatus has been rotated about its axis so that the collimator 202 is positioned at an angle relative to the iso-center of the treatment room. Also shown in the illustration of Figure 6 are a model of the mechanical couch 206 and a target model 204 of a portion of part of the surface of a patient. As is illustrated in Figure 6 the surfaces of the models and in particular the target model surface 204 and adjacent portions of the treatment apparatus 200 and mechanical couch 206 are shown as being texture rendered with a speckled pattern being a projection of a pre-defined speckle pattern projected from a point identified by data within the camera data store 72.

As texture rendering a projected pattern of light onto the surface of a defined computer model can be performed very rapidly, this process can be repeated for multiple models of the patient surface 20, treatment apparatus 16 and mechanical couch 18 as represented by the position instructions in the position instructions store 74.

In some embodiments in addition to generating images based on the projection of a representation of a light pattern onto a model of a patient and a treatment apparatus orientated in particular positions, the simulation apparatus 70 could be configured to generate such images and then process the images to simulate image and/or lens distortions and/or camera mis-calibrations which might be present in a monitoring system.

Having created a set of images of the texture rendered model as viewed from viewpoints corresponding to camera positions as identified by data within the camera data store 72, the simulation apparatus 70 then invokes the 3D position determination module 56 and the model generation module 58 to process 104 the simulated images to generate a model representation of the surface of a patient 204 appearing in the simulated images.

More specifically as when images are processed when monitoring a patient, the 3D position determination module 56 processes images to identify corresponding points in pairs of simulated images of the model from viewpoints corresponding to the positions of cameras in individual camera pods 10 based on the relative positions of corresponding points in obtained pairs of images. The position data generated by the 3D position determination module 56 is then passed to the model generation module 58 which processes the position data to generate a 3D wire mesh model of the surface of a patient 204 appearing in the simulated images. When such a model has been determined it is stored in the generated model store 60.

After models generated based on the simulated images have been stored in the generated model store 60, the model comparison module 78 then proceeds to compare 106 the generated model surfaces with the target model stored in the target model store 62 to determine how the two differ.

As the simulated images generated by the simulation apparatus 70 are created based upon a known defined target model surface any differences between the target model surface and any generated models are representative of errors and uncertainties which arise due to the changing position and location of the patient and the treatment apparatus during treatment, for example due to motion of the gantry and the collimator obscuring the view of the patient from individual camera pods and/or image and/or lens distortions and/or camera mis-calibrations.

A comparison of the generated models and the original target model can therefore highlight portions of the model which may be less reliable as means to monitor the position of a patient 20 or alternatively can highlight expected variations in position which are likely to arise as the modelling software processes images with the patient 20 and treatment apparatus 16 in different positions.

Such measurements can either be used to assist in the identification of the best regions of interest on the surface of a patient 20 which should be monitored or assist with setting boundaries on the limits of acceptable detected motion which arise even if a patient 20 remains entirely motionless on a mechanical couch 18.

Thus for example, a wire mesh model generated for a particular set of simulated images could be generated and compared with the original wire mesh model used to generate the simulated images. A distance measurement could then be determined for each part of the generated model indicative of the distance between that part of the surface and the original model used to generate the simulated images. Where the distance between a generated model surface and the original target model stored in the target model store was greater than a threshold amount that could be taken to indicate that those portions of the generated model are unreliable. The portions of the simulated images which resulted in those parts of the model could then be ignored when generating models in order to monitor the position of a patient 20.

In addition to identifying portions of images which result in the generation of less reliable parts of patient models, a comparison undertaken by the model comparison module 78 could also be utilized to identify the sensitivity of models to the calibration of cameras utilized to monitor a patient.

Thus for example rather than simply generating a single simulated image of a surface of a patient in a particular position from a particular viewpoint, the image generation module 76 could be arranged to generate a set of images from an individual simulated image where the set of images comprise distorted versions of an original simulated image distorted to represent various image and/or lens distortions and/or camera mis-calibrations. Model surfaces could then be generated utilizing the generated sets of images and the variation in the generated model surfaces could then be identified thereby providing an indication of the sensitivity of a monitoring system to distortions arising due to various image and/or lens distortions and/or camera mis-calibrations.

In addition to utilizing the simulation apparatus 70 to identify the sensitivity of a monitoring system to image and/or lens distortions and/or camera mis-calibrations and assisting in identifying portions of images which result in the generation of less reliable surface models, the simulation apparatus 70 could also be utilized to generate images of the patient 20 surface and treatment apparatus 16 in multiple orientations which could then be processed to generate model surfaces of the patient 20 with the patient surface and the treatment apparatus 16 positioned in accordance with positioning instructions in accordance with a particular treatment plan. The differences between such generated models and a target surface orientated in accordance with the positioning instructions could then be utilized to identify how the accuracy of the modelling of the surface of a patient varies during the course of treatment for example due to parts of the surface as viewed from particular viewpoints being obscured by the treatment apparatus. Generating such models could be utilized both to identify less reliable aspects of the models and in addition identifying any expected apparent motion which might occur as model surfaces were built with the patient and monitoring apparatus in different positions.

Thus for example during the course of treatment, as the patient 20 and treatment apparatus 16 are orientated in different positions, this might cause particular parts of a surface model to be generated utilizing data from different camera pods 10 when particular parts of a patient 20 were obscured by the motion of the treatment apparatus 16. Generating simulated models utilizing simulated images of the patient surface and treatment apparatus in different orientations can simulate how the models might be expected to vary over time. The relative positions and orientations of the generated models and the target model surface can be calculated. This can identify portions of the generated models which are less reliable. In addition an offset measurement can be determined by comparing the target model surface with the generated model surface in the same way in which an offset is determined by the matching module 64 of a monitoring system. Thus in this way an expected offset which might be expected to arise with the patient and the treatment apparatus in a particular orientation can be determined. Subsequently when monitoring a patient during treatment the expected apparent motion of a patient 20 could be taken into account when trying to determine whether or not a patient 20 has actually moved out of position.

Although in the above described embodiment a simulation apparatus 70 has been described in which a model is generated based on the identification of corresponding portions of simulated images of the projection of a speckle pattern of light onto a surface of a patient, it will be appreciated that the above described invention could be applied to the determination of errors arising in other forms of patient monitoring system.

Thus for example, as noted previously in some patient monitoring systems rather than matching points on the surface of a patient onto which a speckle pattern is projected, the position of a patient 20 is determined by measuring the deformation of a pattern of structured light such as a line or grid pattern or other pattern of structured light projected onto the surface of a patient 20. Potential modelling errors in such systems could be identified by adapting the approach described above by generating simulated images of the projection of patterns of such structured light onto a target model surface, modelling the surface on the basis of the appearance of the projected pattern in the simulated images and comparing the generated models with a target model utilized to generate the simulated images.

Although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source or object code or in any other form suitable for use in the implementation of the processes according to the invention. The carrier can be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal which may be conveyed via electrical or optical cable or by radio or other means. When a program is embodied in a signal which may be conveyed directly by a cable or other device or means, the carrier may be constituted by such cable or other device or means. Alternatively, the carrier may be an integrated circuit in which the program is embedded, the integrated circuit being adapted for performing, or for use in the performance of, the relevant processes.

## Claims

1. A method of measuring the accuracy of a model of a patient generated by a patient modelling system operable to generate a model of a patient on the basis of images of patterns of light projected onto the surface of a patient, the method comprising:
generating a simulated image of a surface of a patient onto which a pattern of light is projected by texture rendering a model of a surface of a patient;
processing the simulated image to generate a model of the surface of a patient; and
comparing the generated model of the surface of a patient with the model of the surface of the patient utilized to generate the simulated image; and
identifying portions of the simulated image corresponding to more accurate portions of a generated model and utilizing the identified portions to monitor the positioning of a patient.

2. The method of claim 1 wherein generating a simulated image of a surface of a patient onto which a pattern of light is projected by texture rendering a model of a surface of a patient comprises generating a plurality of simulated images of a surface of a patient onto which a pattern of light is projected by texture rendering a model of a surface of a patient.

3. The method of claim 2 comprising generating a plurality of simulated images of a model of a surface of a patient from a plurality of different view points and processing the simulated images to identify corresponding portions of the simulated surface of a patient appearing in the simulated images.

4. The method of claim 2 or 3 wherein the projected pattern of light comprises a speckled pattern of light and generating a simulated image of a surface of a patient onto which a pattern of light is projected by texture rendering a model of a surface of a patient by simulating projecting a speckled pattern of light onto said model.

5. The method of claim 2 comprising generating a simulated image of a surface of a patient onto which a pattern of structured light is projected and processing the simulated image to determine the deformation of the projected pattern of structured light appearing in the simulated image.

6. The method of claim 5 wherein the pattern of structured light comprises a grid pattern or a line of laser light.

7. The method of any preceding claim wherein generating a simulated image of a surface of a patient onto which a pattern of light is projected by texture rendering a model of a surface of a patient further comprises generating a model of a treatment apparatus positioned in accordance with positioning instructions wherein generating a simulated image of a surface of a patient onto which a pattern of light is projected comprises generating a simulated image of a surface of a patient and the treatment apparatus positioned in accordance with positioning instructions onto which a pattern of light is projected.

8. The method of any preceding claim wherein comparing a generated model of the surface of a patient with the model of the surface of the patient utilized to generate the simulated image comprises determining an offset value indicative of an implied distance between a generated model and a model utilized to generate a simulated image.

9. The method of any preceding claim wherein generating a simulated image of a surface of a patient onto which a pattern of light is projected by texture rendering a model of a surface of a patient further comprises manipulating a generated simulated image to represent image and/or lens distortions and/or camera mis-calibrations and utilizing the manipulated simulated image to generate a model of the surface of a patient.

10. A method for measuring the accuracy of a model of a patient generated by a patient modelling system operable to generate a model of a patient on the basis of images of patterns of light projected onto the surface of a patient, the method comprising
generating a simulated image of a surface of a patient onto which a pattern of light is projected by texture rendering a target model of a surface of a patient;
using a modelling system to process the simulated image to generate a model of the surface of a patient;
determining the offset between the target model and the model generated using the simulated image;
obtaining an image of a surface of a patient onto which a pattern of light is projected; using the modelling system to process the obtained image to generate a model of the surface of a patient;
determining the offset between the target model and the model generated using the obtained image; and
comparing the determined offsets determined using the simulated image and the obtained image.

11. An apparatus for measuring the accuracy of a model of a patient generated by a patient modelling system operable to generate a model of a patient on the basis of images of patterns of light projected onto the surface of a patient, the apparatus comprising:
a target model store operable to store a computer model of a surface of a patient;
an image generation module operable to generate simulated images of a surface of a patient onto which a pattern of light is projected by texture rendering a model of a surface of a patient stored in the target model store;
a model generation module operable to process obtained images of a patient and
simulated images of patient generated by the image generation module to generate models of the surface of a patient appearing in the obtained or simulated images; and
a comparison module operable to determine offsets between model surfaces generated by the model generation module and the target model stored in the target model store and compare determined offsets between generated models and target models for models generated based on obtained images and simulated images.

12. The apparatus of claim 11 wherein the image generation module is operable to generate simulated images of a surface of a patient and a treatment apparatus positioned in accordance with positioning instructions onto which a pattern of light is projected by texture rendering a model of a surface of a patient stored in the target model store.

13. The apparatus of claim 11 or 12 wherein the image generation module is operable to generate simulated images of a surface of a patient by manipulating a generated simulated image to represent image and/or lens distortions and/or camera mis-calibrations.

14. A computer readable medium storing instructions which when interpreted by a programmable computer cause the programmable computer to perform the features of claim 1.

## Patentansprüche

1. Verfahren zum Messen der Genauigkeit eines Modells eines Patienten, das von einem Patientenmodellierungssystem erzeugt wird, das dazu betrieben werden kann, ein Modell eines Patienten auf der Grundlage von Bildern von Lichtmustern, die auf die Oberfläche eines Patienten projiziert werden, zu erzeugen, wobei das Verfahren Folgendes umfasst:
Erzeugen eines simulierten Bildes einer Oberfläche eines Patienten, auf die ein Lichtmuster durch Textur-Rendering eines Modells einer Oberfläche eines Patienten projiziert wird;
Verarbeiten des simulierten Bildes, um ein Modell der Oberfläche eines Patienten zu erzeugen; und
Vergleichen des erzeugten Modells der Oberfläche des Patienten mit dem Modell der Oberfläche des Patienten, das dazu genutzt wird, das simulierte Bild zu erzeugen; und
Identifizieren von Abschnitten des simulierten Bildes entsprechend genaueren Abschnitten eines erzeugten Modells und Nutzen der identifizieren Abschnitte, um die Positionierung eines Patienten zu überwachen.

2. Verfahren nach Anspruch 1, wobei das Erzeugen eines simulierten Bildes einer Oberfläche eines Patienten, auf die ein Lichtmuster durch Textur-Rendering eines Modells einer Oberfläche eines Patienten projiziert wird, ein Erzeugen einer Vielzahl von simulierten Bildern einer Oberfläche eines Patienten, auf die ein Lichtmuster durch Textur-Rendering eines Modells einer Oberfläche eines Patienten projiziert wird, umfasst.

3. Verfahren nach Anspruch 2, umfassend ein Erzeugen einer Vielzahl von simulierten Bildern eines Modells einer Oberfläche eines Patienten aus einer Vielzahl von unterschiedlichen Blickpunkten und ein Verarbeiten der simulierten Bilder, um entsprechende Abschnitte der simulierten Oberfläche eines Patienten, die in dem simulierten Bildern erscheinen, zu identifizieren.

4. Verfahren nach Anspruch 2 oder 3, wobei das projizierte Lichtmuster ein gesprenkeltes Lichtmuster und ein Erzeugen eines simulierten Bildes einer Oberfläche eines Patienten, auf die ein Lichtmuster durch Textur-Rendering eines Modells einer Oberfläche eines Patienten projiziert wird, durch Simulieren des Projizierens eines gesprenkelten Lichtmusters auf das Modell umfasst.

5. Verfahren nach Anspruch 2, umfassend ein Erzeugen eines simulierten Bildes einer Oberfläche eines Patienten, auf die ein Muster von strukturiertem Licht projiziert wird, und ein Verarbeiten des simulierten Bildes, um die Deformation des projizierten Musters von strukturiertem Licht, das in dem simulierten Bild erscheint, zu bestimmen.

6. Verfahren nach Anspruch 5, wobei das Muster von strukturiertem Licht ein Gittermuster oder eine Linie aus Laserlicht umfasst.

7. Verfahren nach einem der vorhergehenden Ansprüche, wobei das Erzeugen eines simulierten Bildes einer Oberfläche eines Patienten, auf die ein Lichtmuster durch Textur-Rendering eines Modells einer Oberfläche eines Patienten projiziert wird, ferner ein Erzeugen eines Modells einer Behandlungsvorrichtung, die in Übereinstimmung mit Positionierungsanweisungen positioniert ist, umfasst, wobei das Erzeugen eines simulierten Bildes einer Oberfläche eines Patienten, auf die ein Lichtmuster projiziert wird, ein Erzeugen eines simulierten Bildes einer Oberfläche eines Patienten und der Behandlungsvorrichtung, die in Übereinstimmung mit Positionierungsanweisungen positioniert ist, auf die ein Lichtmuster projiziert wird, umfasst.

8. Verfahren nach einem der vorhergehenden Ansprüche, wobei das Vergleichen eines erzeugten Modells der Oberfläche eines Patienten mit dem Modell der Oberfläche des Patienten, das dazu genutzt wird, das simulierte Bild zu erzeugen, ein Bestimmen eines Versatzwertes umfasst, der einen implizierten Abstand zwischen einem erzeugten Modell und einem Modell, das dazu genutzt wird, ein simuliertes Bild zu erzeugen, angibt.

9. Verfahren nach einem der vorhergehenden Ansprüche, wobei das Erzeugen eines simulierten Bildes einer Oberfläche eines Patienten, auf die ein Lichtmuster durch Textur-Rendering eines Modells einer Oberfläche eines Patienten projiziert wird, ferner ein Manipulieren eines erzeugten simulierten Bildes, um Bild- und/oder Linsenverzerrungen und/oder Kamerafehlkalibrierungen darzustellen, und ein Nutzen des manipulierten simulierten Bildes, um ein Modell der Oberfläche eines Patienten zu erzeugen, umfasst.

10. Verfahren zum Messen der Genauigkeit eines Modells eines Patienten, das von einem Patientenmodellierungssystem erzeugt wird, das dazu betrieben werden kann, ein Modell eines Patienten auf der Grundlage von Bildern von Lichtmustern, die auf die Oberfläche eines Patienten projiziert werden, zu erzeugen, wobei das Verfahren Folgendes umfasst:
Erzeugen eines simulierten Bildes einer Oberfläche eines Patienten, auf die ein Lichtmuster durch Textur-Rendering eines Zielmodells einer Oberfläche eines Patienten projiziert wird;
Verwenden eines Modellierungssystems, um das simulierte Bild zu verarbeiten, um ein Modell der Oberfläche eines Patienten zu erzeugen;
Bestimmen des Versatzes zwischen dem Zielmodell und dem Modell, das unter Verwendung des simulierten Bildes erzeugt wird;
Erhalten eines Bildes einer Oberfläche eines Patienten, auf die ein Lichtmuster projiziert wird;
Verwenden des Modellierungssystems, um das erhaltene Bild zu verarbeiten, um ein Modell der Oberfläche eines Patienten zu erzeugen;
Bestimmen des Versatzes zwischen dem Zielmodell und dem Modell, das unter Verwendung des erhaltenen Bildes erzeugt wird; und
Vergleichen der bestimmten Versätze, die unter Verwendung des simulierten Bildes und des erhaltenen Bildes bestimmt werden.

11. Vorrichtung zum Messen der Genauigkeit eines Modells eines Patienten, das von einem Patientenmodellierungssystem erzeugt wird, das dazu betrieben werden kann, ein Modell eines Patienten auf der Grundlage von Bildern von Lichtmustern, die auf die Oberfläche eines Patienten projiziert werden, zu erzeugen, wobei die Vorrichtung Folgendes umfasst:
einen Zielmodellspeicher, der dazu betrieben werden kann, ein Computermodell einer Oberfläche eines Patienten zu speichern;
ein Bilderzeugungsmodul, das dazu betrieben werden kann, simulierte Bilder einer Oberfläche eines Patienten, auf die ein Lichtmuster durch Textur-Rendering eines Zielmodells einer Oberfläche eines Patienten, das in dem Zielmodellspeicher gespeichert ist, projiziert wird, zu erzeugen;
ein Modellerzeugungsmodul, das dazu betrieben werden kann, erhaltene Bilder eines Patienten und simulierte Bilder eines Patienten, die durch das Bilderzeugungsmodul erzeugt wurden, zu verarbeiten, um Modelle der Oberfläche eines Patienten, die in den erhaltenen oder simulierten Bildern erscheinen, zu erzeugen; und
ein Vergleichsmodul, das dazu betrieben werden kann, Versätze zwischen Modelloberflächen, die durch das Modellerzeugungsmodul erzeugt werden, und dem Zielmodell, das in dem Zielmodellspeicher gespeichert ist, zu bestimmen und bestimmte Versätze zwischen erzeugten Modellen und Zielmodellen für Modelle, die auf Grundlage von erhaltenen Bildern und simulierten Bilden erzeugt werden, zu vergleichen.

12. Vorrichtung nach Anspruch 11, wobei das Bilderzeugungsmodul dazu betrieben werden kann, simulierte Bilder einer Oberfläche eines Patienten und einer Behandlungsvorrichtung, die in Übereinstimmung mit Positionierungsanweisungen positioniert ist, auf die ein Lichtmuster durch Textur-Rendering eines Zielmodells einer Oberfläche eines Patienten, das in dem Zielmodellspeicher gespeichert ist, projiziert wird, zu erzeugen.

13. Vorrichtung nach Anspruch 11 oder 12, wobei das Bilderzeugungsmodul dazu betrieben werden kann, simulierte Bilder einer Oberfläche eines Patienten durch Manipulieren eines erzeugten simulierten Bildes, um Bild- und/oder Linsenverzerrungen und/oder Kamerafehlkalibrierungen darzustellen, zu erzeugen.

14. Computerlesbares Medium, das Anweisungen speichert, die, wenn sie durch einen programmierbaren Computer interpretiert werden, den programmierbaren Computer veranlassen, die Merkmale von Anspruch 1 auszuführen.

## Revendications

1. Procédé de mesure de la précision d'un modèle d'un patient généré par un système de modélisation de patient utilisable pour générer un modèle d'un patient sur la base d'images de motifs de lumière projetés sur la surface d'un patient, le procédé comprenant
la génération d'une image simulée d'une surface d'un patient sur laquelle un motif de lumière est projeté par rendu de texture d'un modèle d'une surface d'un patient ;
le traitement de l'image simulée pour générer un modèle de la surface d'un patient ; et la comparaison du modèle généré de la surface d'un patient avec le modèle de la surface du patient utilisé pour générer l'image simulée ; et
l'identification de parties de l'image simulée correspondant à des parties plus précises d'un modèle généré et l'utilisation des parties identifiées pour surveiller le positionnement d'un patient.

2. Procédé selon la revendication 1, dans lequel la génération d'une image simulée d'une surface d'un patient sur laquelle un motif de lumière est projeté par rendu de texture d'un modèle d'une surface d'un patient comprend la génération d'une pluralité d'images simulées d'une surface d'un patient sur laquelle un motif de lumière est projeté par rendu de texture d'un modèle d'une surface d'un patient.

3. Procédé selon la revendication 2, comprenant la génération d'une pluralité d'images simulées d'un modèle d'une surface d'un patient à partir d'une pluralité de points de vue différents et le traitement des images simulées pour identifier des parties correspondantes de la surface simulée d'un patient apparaissant dans les images simulées.

4. Procédé selon la revendication 2 ou 3, dans lequel le motif de lumière projeté comprend un motif de lumière tacheté et la génération d'une image simulée d'une surface d'un patient sur laquelle un motif de lumière est projeté par rendu de texture d'un modèle d'une surface d'un patient en simulant la projection d'un motif de lumière tacheté sur ledit modèle.

5. Procédé selon la revendication 2, comprenant la génération d'une image simulée d'une surface d'un patient sur laquelle un motif de lumière structurée est projeté et le traitement de l'image simulée pour déterminer la déformation du motif projeté de lumière structurée apparaissant dans l'image simulée.

6. Procédé selon la revendication 5, dans lequel le motif de lumière structurée comprend un motif de grille ou une ligne de lumière laser.

7. Procédé selon l'une quelconque des revendications précédentes, dans lequel la génération d'une image simulée d'une surface d'un patient sur laquelle un motif de lumière est projeté par rendu de texture d'un modèle d'une surface d'un patient comprend en outre la génération d'un modèle d'un appareil de traitement positionné conformément à des instructions de positionnement dans lesquelles la génération d'une image simulée d'une surface d'un patient sur laquelle un motif de lumière est projeté comprend la génération d'une image simulée d'une surface d'un patient et l'appareil de traitement positionné conformément aux instructions de positionnement sur lesquelles un motif de lumière est projeté.

8. Procédé selon l'une quelconque des revendications précédentes, dans lequel la comparaison d'un modèle généré de la surface d'un patient avec le modèle de la surface du patient utilisé pour générer l'image simulée comprend la détermination d'une valeur de décalage indicative d'une distance implicite entre un modèle généré et un modèle utilisé pour générer une image simulée.

9. Procédé selon l'une quelconque des revendications précédentes, dans lequel la génération d'une image simulée d'une surface d'un patient sur laquelle un motif de lumière est projeté par rendu de texture d'un modèle d'une surface d'un patient comprend en outre la manipulation d'une image simulée générée pour représenter des distorsions d'image et/ou de lentille et/ou mauvais calibrages de la caméra et l'utilisation de l'image simulée manipulée pour générer un modèle de la surface d'un patient.

10. Procédé de mesure de la précision d'un modèle d'un patient généré par un système de modélisation de patient utilisable pour générer un modèle d'un patient sur la base d'images de motifs de lumière projetés sur la surface d'un patient, le procédé comprenant la génération d'une image simulée d'une surface d'un patient sur laquelle un motif de lumière est projeté par rendu de texture d'un modèle cible d'une surface d'un patient ; l'utilisation d'un système de modélisation pour traiter l'image simulée afin de générer un modèle de la surface d'un patient ;
la détermination du décalage entre le modèle cible et le modèle généré à l'aide de l'image simulée ;
l'obtention d'une image d'une surface d'un patient sur laquelle un motif de lumière est projeté ;
l'utilisation du système de modélisation pour traiter l'image obtenue afin de générer un modèle de la surface d'un patient ;
la détermination du décalage entre le modèle cible et le modèle généré en utilisant l'image obtenue ; et
la comparaison des décalages déterminés qui ont été déterminés en utilisant l'image simulée et de l'image obtenue.

11. Appareil de mesure de la précision d'un modèle d'un patient généré par un système de modélisation de patient utilisable pour générer un modèle d'un patient sur la base d'images de motifs de lumière projetés sur la surface d'un patient, l'appareil comprenant :
une mémoire de modèle cible utilisable pour mémoriser un modèle informatique d'une surface d'un patient ;
un module de génération d'images utilisable pour générer des images simulées d'une surface d'un patient sur laquelle un motif de lumière est projeté par rendu de texture d'un modèle d'une surface d'un patient mémorisée dans la mémoire de modèle cible ;
un module de génération de modèle utilisable pour traiter des images obtenues d'un patient et des images simulées de patient générées par le module de génération d'images pour générer des modèles de la surface d'un patient apparaissant dans les images obtenues ou simulées ; et
un module de comparaison utilisable pour déterminer des décalages entre des surfaces de modèle générées par le module de génération de modèle et le modèle cible mémorisé dans la mémoire de modèle cible et comparer des décalages déterminés entre des modèles générés et des modèles cibles pour des modèles générés sur la base d'images obtenues et d'images simulées.

12. Appareil selon la revendication 11, dans lequel le module de génération d'images est utilisable pour générer des images simulées d'une surface d'un patient et un appareil de traitement positionné conformément à des instructions de positionnement sur lesquelles un motif de lumière est projeté par rendu de texture d'un modèle d'une surface d'un patient mémorisé dans la mémoire de modèle cible.

13. Appareil selon la revendication 11 ou 12, dans lequel le module de génération d'images est utilisable pour générer des images simulées d'une surface d'un patient en manipulant une image simulée générée pour représenter des distorsions d'image et/ou de lentille et/ou des mauvais étalonnages de caméra.

14. Support lisible par ordinateur stockant des instructions qui, lorsqu'elles sont interprétées par un ordinateur programmable, amènent l'ordinateur programmable à exécuter les caractéristiques de la revendication 1.
